# EUROPÄISCHE PATENTSCHRIFT

(11) **EP 0 680 468 B1**
(45) Veröffentlichungstag und Bekanntmachung des Hinweises auf die Patenterteilung: **24.06.1998**
(21) Anmeldenummer: 94905012.4
(22) Anmeldetag: 07.01.1994
(51) Int. Cl.: C07C 275/24, C07D 233/76, C07C 271/22, C07C 279/18, A61K 31/17, A61K 31/27, A61K 31/415, A61K 31/155

(54) **SUBSTITUIERTE AMINOVERBINDUNGEN, IHRE HERSTELLUNG UND IHRE VERWENDUNG ALS HEMMSTOFFE DER THROMBOZYTENAGGREGATION**
SUBSTITUTED AMINO COMPOUNDS, THEIR PREPARATION AND THEIR USE AS INHIBITORS OF THROMBOCYTE AGGREGATION
COMPOSES AMINO SUBSTITUES, LEUR FABRICATION ET LEUR UTILISATION COMME AGENTS INHIBITEURS DE L'AGREGATION DE THROMBOCYTES

(30) Priorität: 23.01.1993 DE 4301747
(43) Veröffentlichungstag der Anmeldung: 08.11.1995
(73) Patentinhaber: HOECHST AKTIENGESELLSCHAFT, 65926 Frankfurt am Main (DE)
(72) Erfinder: ZOLLER, Gerhard, D-61137 Schöneck (DE); JABLONKA, Bernd, D-65812 Bad Soden (DE); JUST, Melitta, D-63225 Langen (DE); KLINGLER, Otmar, D-63110 Rodgau (DE); BREIPOHL, Gerhard, D-60529 Frankfurt am Main (DE); KNOLLE, Jochen, D-65830 Kriftel (DE); KÖNIG, Wolfgang, D-94375 Stallwang (DE)
(86) Internationale Anmeldenummer: EP9400029
(87) Internationale Veröffentlichungsnummer: WO9417034

(56) Entgegenhaltungen:
- EP-A- 0 352 249
- EP-A- 0 445 796
- EP-A- 0 449 079
- EP-A- 0 513 810
- CHEMICAL ABSTRACTS, vol. 101, no. 1, 2. Juli 1984, Columbus, Ohio, US; abstract no. 7605p, E. P. HEIMER ET AL. 'Synthesis of analogs and oligomers of N-(2-aminoethyl)glycine and their gastrointestinal absorption in the rat' Seite 656 ; & INT. J. PEPT. PROTEIN RES. Bd. 23, Nr. 2 , 1984 Seiten 203 - 211

## Beschreibung

Die vorliegende Erfindung betrifft substituierte Aminoverbindungen, ihre Herstellung und ihre Anwendung als Hemmstoffe der Blutplättchenaggregation.

In Int. J. Peptide Protein Res., Band 23 (1984), Seite 203 (Chemical Abstracts, Band 101, Abstract Nr. 7605p) sind bestimmte Aminoverbindungen beschrieben, die sich aber von den Verbindungen der vorliegenden Erfindung strukturell unterscheiden und für die keine pharmakologische Wirksamkeit beschrieben wird.

In der EP-A 449 079 sind Hydantoinderivate beschrieben, die thrombozytenaggregationshemmende Wirkungen aufweisen. Weitere Forschungsarbeiten haben die Verbindungen der vorliegenden Erfindung als weitere starke Hemmstoffe der Blutplättchenaggregation bereitgestellt.

Die vorliegende Erfindung betrifft Verbindungen der allgemeinen Formel I,

R¹-(A)ₐ-(B)_{b}-(D)_{c}-(CH₂)ₘ-N(R²)-(CH₂)ₙ-R³ (I)

worin
- A: einen zweiwertigen Rest aus der Reihe Methylen, Ethylen, Trimethylen, Tetramethylen, Cyclohexylen, Phenylen, Phenylenmethyl, Phenylenethenyl und Phenylenmethylen bedeutet;
- B: einen zweiwertigen Rest aus der Reihe Methylen, Ethylen, Trimethylen, Tetramethylen, Vinylen, Phenylen, 2,5-Dioxo-imidazolidin-1,4-diyl, 5-Oxo-2-thioxoimidazolidin-1,4-diyl, 2,5-Dioxo-pyrrolidin-1,4-diyl und 2,4-Dioxo-oxazolidin-3,5-diyl bedeutet;
- D: einen zweiwertigen Rest aus der Reihe Carbonylimino, Iminocarbonyl und Iminocarbonylimino bedeutet;
- R¹: -CH₂-NH-X, -C(=NH)-NH-X¹ oder -NH-C(=NX¹)-NH-X^{1'} bedeutet;
- X, X¹ und X^{1'}: unabhängig voneinander Wasserstoff, (C₁-C₆)-Alkylcarbonyl, (C₁-C₆)-Alkoxycarbonyl oder (C₁-C₈)-Alkylcarbonyloxy-(C₁-C₆)-alkoxycarbonyl bedeuten;
- R²: -COR⁴, -COOR⁴, -CO-NH-R⁴ oder -CS-NH-R⁴ bedeutet;
- R³: -COOH oder -COO-(C₁-C₁₈)-Alkyl bedeutet;
- R⁴: (C₁-C₆)-Alkyl oder (C₆-C₁₄)-Aryl-(C₁-C₈)-alkyl bedeutet oder im Falle, daß R² für -CO-NH-R⁴ oder -CSNH-R⁴ steht, -NH-R⁴ für einen α-Aminosäurerest oder dessen ω-Amino-(C₂-C₈)-alkylamid steht;
- a, b und c: 0 oder 1 bedeuten, aber nicht alle gleichzeitig 0 sein können; und
- m: für 2 oder 3 und n für 1 steht;
sowie deren physiologisch verträgliche Salze.

Alkylreste können geradkettig oder verzweigt sein. Bevorzugte Alkylreste sind Methyl, Ethyl, n-Propyl, i-Propyl, n-Butyl, i-Butyl, sek-Butyl und tert.-Butyl. Entsprechendes gilt für Reste wie Alkylen, Alkoxy, Alkoxycarbonyl oder Aralkyl.

Ein Cyclohexylenrest kann auch durch beispielsweise (C₁-C₄)-Alkyl substituiert sein.

(C₆-C₁₄)-Arylgruppen sind beispielsweise Phenyl, Naphthyl, Biphenylyl oder Fluorenyl, wobei Phenyl und Naphthyl bevorzugt sind. Entsprechendes gilt für Aralkylreste. Aralkylreste sind insbesondere Benzyl sowie 1- und 2-Naphthylmethyl, die auch substituiert sein können. Substituierte Aralkylreste sind beispielsweise Halobenzyl oder (C₁-C₄)-Alkoxybenzyl.

Ist Phenyl zweifach substituiert, können die Substituenten in 1,2-, 1,3- oder 1,4-Position zueinander stehen. Die 1,3- sowie die 1,4-Position sind bevorzugt.

Unter dem Rest Phenylenmethylen ist ein Phenylen-(C₁)-alkyliden-Rest zu verstehen.

Halogen steht für Fluor, Chlor, Brom oder Jod, insbesondere für Fluor oder Chlor.

Natürliche und unnatürliche α-Aminosäuren können, falls chiral, in der D- oder L-Form vorliegen. Beispielsweise seien genannt (vgl. Houben-Weyl, Methoden der organischen Chemie, Band XV/1 und 2, Stuttgart, 1974):
Aad, Abu, εAca, Ach, Acp, Aib, Ala, ΔAla, Alg, All, Ama, Apm, Apr, Arg, Asn, Asp, Asu, Aze, Azi, Bph, Can, Cit, Cys, (Cys)₂, Cyta, Daad, Dab, Dadd, Dap, Dapm, Dasu, Djen, Dpa, Dtc, Fel, Gln, Glu, Gly, hAla, hArg, hCys, hGln, hGlu, His, hlle, hLeu, hLys, hMet, hPhe, hPro, hSer, hThr, hTrp, hTyr, Hyl, Hyp, 3Hyp, lle, Iva, Lant, Lcn, Leu, Lys, ΔLys, Met, Min, nArg, Nle, Nva, Oly, Orn, Pan, Pec, Pen, Phe, Phg, Pic, Pro, ΔPro, Pse, Pya, Pyr, Pza, Sar, Sec, Sem, Ser, Thi, Thr, Thy, Thx, Tia, Tle, Tly, Trp, Trta, Tyr, Val, Tbg, Npg, Chg, Cha, Thia, 2,2-Diphenylaminoessigsäure, 2-(p-Tolyl)-2-phenylaminoessigsäure, 2-(p-Chlorphenyl)-aminoessigsäure.

Aminosäuren können auch als Ester bzw. Amide vorliegen, wie z.B. Methylester, Ethylamid, Semicarbazid oder ω-Amino-(C₄-C₈)-alkylamid.

Funktionelle Gruppen der Aminosäuren können geschützt vorliegen. Geeignete Schutzgruppen wie z.B. Urethanschutzgruppen, Carboxylschutzgruppen und Seitenkettenschutzgruppen sind bei Hubbuch, Kontakte (Merck) 1979, Nr. 3, Seiten 14 bis 23 und bei Büllesbach, Kontakte (Merck) 1980, Nr. 1, Seiten 23 bis 35 beschrieben. Insbesondere seien genannt: Aloc, Pyoc, Fmoc, Tcboc, Z, Boc, Ddz, Bpoc, Adoc, Msc, Moc, Z(NO₂), Z(Halₙ), Bobz, Iboc, Adpoc, Mboc, Acm, tert.-Butyl, OBzl, ONbzl, OMbzl, Bzl, Mob, Pic, Trt.

Physiologisch verträgliche Salze der Verbindungen der allgemeinen Formel I sind insbesondere pharmazeutisch verwendbare oder nicht-toxische Salze.

Solche Salze werden beispielsweise von Verbindungen der allgemeinen Formel I, welche saure Gruppen, z.B. Carboxy, enthalten, mit Alkali- oder Erdalkalimetallen gebildet, wie z.B. Na, K, Mg und Ca, sowie mit physiologisch verträglichen organischen Aminen, wie z. B. Triethylamin und Tris-(2-hydroxy-ethyl)-amin.

Verbindungen der allgemeinen Formel I, welche basische Gruppen, z.B. eine Aminogruppe oder eine Guanidinogruppe enthalten, bilden mit anorganischen Säuren, wie z.B. Salzsäure, Schwefelsäure oder Phosphorsäure und mit organischen Carbon- oder Sulfonsäuren, wie z.B. Essigsäure, Citronensäure, Benzoesäure, Maleinsäure, Fumarsäure, Weinsäure und p-Toluolsulfonsäure Salze.

Die erfindungsgemäßen Verbindungen der allgemeinen Formel I können optisch aktive Kohlenstoffatome enthalten und somit in Form reiner Enantiomerer oder in Form von Enantiomerengemischen vorliegen. Sowohl reine Enantiomere als auch Enantiomerengemische sind Gegenstand der vorliegenden Erfindung.

Die erfindungsgemäßen Verbindungen der allgemeinen Formel I können darüber hinaus bewegliche Wasserstoffatome enthalten, also in verschiedenen tautomeren Formen vorliegen. Auch diese Tautomeren sind Gegenstand der vorliegenden Erfindung.

Ein für -NH-R⁴ stehender α-Aminosäurerest ist besonders bevorzugt der Valin-, Lysin-, Phenylalanin-, Tryptophan- oder Phenylglycin-Rest.

Ein besonders bevorzugtes ω-Amino-(C₂-C₈)-alkylamid ist das 4-Aminobutylamid.

Die erfindungsgemäßen Verbindungen der allgemeinen Formel I können hergestellt werden durch Fragmentkondensation einer Verbindung der allgemeinen Formel II

R¹-(A)ₐ-(B)_{b}-Y (II)

mit einer Verbindung der allgemeinen Formel III

Z-(CH₂)ₘ-N(R²)-(CH₂)ₙ-R³ (III)

worin a, b, m und n sowie die Reste A, B und R¹ bis R³ wie oben angegeben definiert sind und Y für Hydroxycarbonyl, Alkoxycarbonyl oder ein aktiviertes Carbonsäurederivat, wie ein Säurechlorid oder einen Aktivester, und Z für Amino steht oder worin Y für Amino und Z für Hydroxycarbonyl, Alkoxycarbonyl oder ein aktiviertes Carbonsäurederivat steht.

Zur Kondensation der Verbindungen der allgemeinen Formel II mit denen der allgemeinen Formel III verwendet man vorteilhafterweise die an sich bekannten Methoden der Peptidchemie (siehe z.B. Houben Weyl, Methoden der organischen Chemie, Band 15/1 und 15/2, Stuttgart, 1974).

Dazu ist es in der Regel nötig, daß in R¹ und R⁴ enthaltene Aminogruppen durch reversible Schutzgruppen geschützt werden. Gleiches gilt für die Carboxylgruppen der Verbindung der allgemeinen Formel III, die bevorzugt als (C₁-C₆)-Alkyl, Benzyl- oder tert.-Butylester vorliegen. Ein Aminogruppen-Schutz erübrigt sich, wenn die zu generierenden Aminogruppen als Nitro- oder Cyanogruppen vorliegen und erst nach der Kupplung durch Hydrierung gebildet werden.

Nach der Kupplung werden die vorhandenen Schutzgruppen in geeigneter Weise abgespalten. Beispielsweise können NO₂-Gruppen (Guanidinoschutz), Benzyloxycarbonylgruppen und Benzylester abhydriert werden. Schutzgruppen vom tert.-Butyltyp werden sauer gespalten, während der 9-Fluorenylmethyloxycarbonylrest durch sekundäre Amine entfernt wird.

Verbindungen der allgemeinen Formel I, bei denen B für einen oxo- oder thioxo-substituierten Imidazolidinring stehen, können auch durch Reaktion von Aminosäuren, N-Alkylaminosäuren oder bevorzugt deren Ester (beispielsweise Methyl-, Ethyl-, Benzyl- oder tert.-Butylester) mit einem Isocyanat oder einem Isothiocyanat und anschließendem Erhitzen des entstandenen Harnstoff- oder Thioharnstoffderivats mit Säure hergestellt werden. Dabei erfolgt gleichzeitig eine Verseifung der Esterfunkion.

Beispielsweise kann eine Verbindung der allgemeinen Formel IV

R¹-(A)ₐ-CH(NH₂)-COOCH₃ (IV)

mit einer Verbindung der allgemeinen Formel V

W=C=N-(CH₂)ₘ-N(R²)-(CH₂)ₙ-R³ (V)

worin A, a, R¹, R², R³, m und n wie oben angegeben definiert sind, und W Sauerstoff oder Schwefel bedeutet, zu einer Verbindung der allgemeinen Formel VI

R¹-(A)ₐ-CH(COOCH₃)-NH-C(=W)-NH-(CH₂)ₘ-N(R²)-(CH₂)ₙ-R³ (VI)

umgesetzt werden. Letztere kann durch Erhitzen mit Säure unter Verseifung der Esterfunktionen zu einer Verbindung der allgemeinen Formel Ia 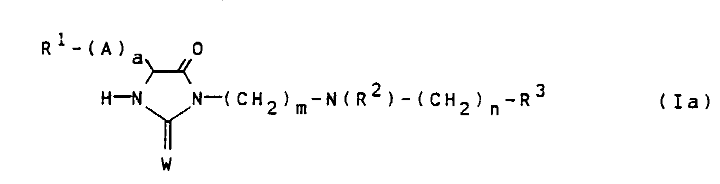 cyclisiert werden.

Alternativ können Verbindungen der allgemeinen Formel Ia durch Umsetzung von Verbindungen der allgemeinen Formel VII 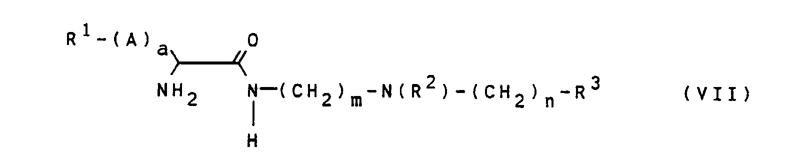 mit Phosgen, Thiophosgen oder entsprechenden Äquivalenten zu den Estern der Imidazolidinderivate und anschließende Verseifung zu den Carbonsäuren erhalten werden (analog S.Goldschmidt, M.Wich, Liebigs Ann.Chem.575 (1952) 217-231; C.Tropp, Chem.Ber.61, (1928) 1431-1439).

Die Guanylierung der Aminofunktion kann mit folgenden Reagentien durchgeführt werden:
1. O-Methylisoharnstoff (S. Weiss und H. Krommer, Chemiker Zeitung 98 (1974) 617 - 618),
2. S-Methylisothioharnstoff (R. F. Borne, M. L. Forrester und I. W. Waters, J. Med. Chem. 20 (1977) 771 - 776),
3. Nitro-S-Methylisothioharnstoff (L. S. Hafner und R. E. Evans, J. Org. Chem. 24 (1959) 1157),
4. Formamidinosulfonsäure (K. Kim, Y.-T. Lin und H. S. Mosher, Tetrah. Lett. 29 (1988) 3183 - 3186),
5. 3.5-Dimethyl-1-pyrazolyl-formamidinium-nitrat (F. L. Scott, D. G. O'Donovan und J. Reilly, J. Amer. Chem. Soc. 75 (1953) 4053 - 4054).
6. N,N'-Di-tert.-butyloxycarbonyl-S-methyl-isothioharnstoff (R. J. Bergeron und J. S. McManis, J. Org. Chem. 52 (1987) 1700- 1703).
7. N-Alkoxycarbonyl-, N,N'-Dialkoxycarbonyl-, N-Alkylcarbonyl- und N,N'-Dialkylcarbonyl-S-methyl-isothioharnstoff (H. Wollweber, H. Kölling, E. Niemers, A. Widding, P. Andrews, H.-P. Schulz und H. Thomas, Arzneim. Forsch./ Drug Res. 34 (1984) 531 - 542).

Formamidine können aus den entsprechenden Cyanoverbindungen durch Anlagerung von Alkoholen (z. B. Methanol oder Ethanol) in saurem wasserfreiem Medium (z. B. Dioxan, Methanol oder Ethanol) und anschließender Behandlung mit Ammoniak in Alkoholen (z. B. Isopropanol, Methanol oder Ethanol) hergestellt werden (G. Wagner, P. Richter und Ch. Garbe, Pharmazie 29 (1974) 12 - 15). Eine weitere Methode, Formamidine herzustellen, ist die Anlagerung von H₂S an die Cyanogruppe, gefolgt von einer Methylierung des entstandenen Thioamids und anschließender Umsetzung mit Ammoniak (DDR-Patent Nr. 235 866).

Die Verbindungen der allgemeinen Formel I und ihre physiologisch verträglichen Salze können als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der allgemeinen Formel I oder eines Salzes davon, neben üblichen pharmazeutisch einwandfreien Träger- und Zusatzstoffen enthalten. Die Zubereitungen enthalten normalerweise etwa 0,5 bis 90 Gew.% der therapeutisch wirksamen Verbindung.

Die Heilmittel können oral, z. B. in Form von Pillen, Tabletten, Lacktabletten, Dragees, Granulaten, Hart- und Weichgelatinekapseln, Lösungen, Sirupen, Emulsionen oder Suspensionen oder Aerosolmischungen verabreicht werden. Die Verabreichung kann aber auch rektal, z. B. in Form von Suppositorien oder parenteral, z. B. in Form von Injektionslösungen, Mikrokapseln oder Rods, perkutan, z. B. in Form von Salben oder Tinkturen, oder nasal, z. B. in Form von Nasalsprays, erfolgen.

Die Herstellung der pharmazeutischen Präparate erfolgt in an sich bekannter Weise, wobei pharmazeutisch inerte anorganische oder organische Trägerstoffe verwendet werden. Für die Herstellung von Pillen, Tabletten, Dragees und Hartgelatinekapseln kann man z. B. Lactose, Maisstärke oder Derivate davon, Talk, Stearinsäure oder deren Salze etc. verwenden. Trägerstoffe für Weichgelatinekapseln und Suppositorien sind z. B. Fette, Wachse, halbfeste und flüssige Polyole, natürliche oder gehärtete Öle etc. Als Trägerstoffe für die Herstellung von Lösungen und Sirupen eignen sich z. B. Wasser, Saccharose, Invertzucker, Glukose, Polyole etc. Als Trägerstoffe für die Herstellung von Injektionslösungen eignen sich Wasser, Alkohole, Glycerin, Polyole, pflanzliche Öle etc. Als Trägerstoffe für Mikrokapseln, Implantate oder Rods eignen sich Mischpolymerisate aus Glykolsäure und Milchsäure.

Die pharmazeutischen Präparate können neben den Wirk- und Trägerstoffen noch Zusatzstoffe, wie z. B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-, Dickungs-, Verdünnungsmittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der allgemeinen Formel I oder ihrer physiologisch verträglichen Salze und noch einen oder mehrere andere therapeutisch wirksame Stoffe enthalten.

Derartige andere therapeutisch wirksame Substanzen sind beispielsweise durchblutungsfördernde Mittel, wie Dihydroergocristin, Nicergolin, Buphenin, Nicotinsäure und ihre Ester, Pyridylcarbinol, Bencyclan, Cinnarizin, Naftidrofuryl, Raubasin und Vincamin; positiv inotrope Verbindungen, wie Digoxin, Acetyldigoxin, Metildigoxin und Lanato-Glykoside; Coronardilatatoren, wie Carbochromen; Dipyridamol, Nifedipin und Perhexilin; antianginöse Verbindungen, wie Isosorbiddinitrat, Isosorbidmononitrat, Glycerolnitrat, Molsidomin und Verapamil; β-Blocker, wie Propranolol, Oxprenolol, Atenolol, Metoprolol und Penbutolol. Darüberhinaus lassen sich die Verbindungen mit anderen nootrop wirksamen Substanzen, wie z. B. Piracetam, oder ZNS-aktiven Substanzen, wie Pirlindol, Sulpirid etc., kombinieren.

Die Dosis kann innerhalb weiter Grenzen variieren und ist in jedem einzelnen Fall den individuellen Gegebenheiten anzupassen. Im allgemeinen ist bei der oralen Verabreichung eine Tagesdosis von etwa 0,1 bis 1 mg/kg, vorzugsweise 0,3 bis 0,5 mg/kg Körpergewicht zur Erzielung wirksamer Ergebnisse angemessen, bei intravenöser Applikation beträgt die Tagesdosis im allgemeinen etwa 0,01 bis 0,3 mg/kg, vorzugsweise 0,05 bis 0,1 mg/kg Körpergewicht. Die Tagesdosis wird normalerweise, insbesondere bei der Applikation größerer Mengen, in mehrere, z. B. 2, 3 oder 4 Teilverabreichungen aufgeteilt. Gegebenenfalls kann es, je nach individuellem Verhalten, erforderlich werden, von der angegebenen Tagesdosis nach oben oder nach unten abzuweichen. Pharmazeutische Präparate enthalten normalerweise 0,2 bis 50 mg, vorzugsweise 0,5 bis 10 mg Wirkstoff der allgemeinen Formel I oder eines ihrer physiologisch verträglichen Salze pro Dosis.

Die erfindungsgemäßen Verbindungen der Formel I haben die Fähigkeit, die Zell-Zell-Adhäsion zu hemmen, die auf der Interaktion von Arg-Gly-Asp-enthaltenden Proteinen, wie Fibronectin, Fibrinogen oder des von Willebrand-Faktors mit den sogenannten Integrinen beruht. Integrine sind Transmembran-Glykoproteine, Rezeptoren für Arg-Gly-Asp-enthaltende Zellmatrix-Glykoproteine (E. Ruoslahti und M. D. Pierschbacher, Science 238 (1987) 491 - 497; D. R. Phillips, I. F. Charo, L. V. Parise und L. A. Fitzgerald, Blood 71 (1988) 831 - 843). Außerdem hemmen sie die Bindung weiterer adhäsiver Proteine, wie Vitronectin, Kollagen und Laminin an die entsprechenden Rezeptoren auf der Oberfläche verschiedener Zelltypen.

Die erfindungsgemäßen Verbindungen der allgemeinen Formel I hemmen die Thrombozytenaggregation, die Metastasierung von Karzinomzellen sowie die Osteoclastenbindung an die Knochenoberflächen.

Die Verbindungen der allgemeinen Formel I finden akut Anwendung bei Thrombosegefahr und chronisch bei der Prävention der Arteriosklerose und Thrombose, z. B. bei der Therapie und Prophylaxe arterieller Gefäßerkrankungen, wie bei akutem Myokardinfarkt, Sekundärprävention des Myokardinfarkts, Reokklusionsprophylaxe nach Lyse und Dilatation (PTCA), instabiler Angina pectoris, transitorischen ischämischen Attacken, Schlaganfall, koronarer Bypass-Operation einschließlich Reokklusionsprophylaxe bei Bypass, Lungenembolie, peripherer arterieller Verschlußkrankheit, dissezierendem Aneurysma; bei der Therapie venöser und mikrozirkulatorischer Gefäßerkrankungen, wie tiefer Venenthrombose, disseminierter intravaskulärer Gerinnung, postoperativem und post-partum Trauma, chirurgischem oder infektiösem Schock, Septicämie oder bei Erkrankungen mit hyperreagiblen Thrombozyten, thrombotischer thrombozytopenischer Purpura, Preeklampsie, prämenstruellem Syndrom, Dialyse oder extrakorporaler Zirkulation; eine weitere Anwendung ist während Krebsoperationen und auch prophylaktisch bei Krebs gegeben. Ferner kann Osteoporose durch Hemmung der Osteoclastenbindung an die Knochenoberfläche verhindert werden.

Geprüft werden die Verbindungen vor allem auf ihre hemmende Wirkung bei der Blutplättchenaggregation und der Anhaftung von Fibrinogen an Blutplättchen (verwendet werden gelfiltrierte Blutplättchen aus humanem Spenderblut, die mit ADP oder Thrombin aktiviert werden), sowie auf ihre in vivo-Wirkung zur Hemmung der Thrombozytenaggregation und Thrombosehemmung.

Geprüft wird die Hemmung der Bindung von Fibrinogen an seinen Rezeptor (Glykoprotein IIb/IIIa) an intakten, gelfiltrierten Human-Thrombozyten durch die erfindungsgemäßen Verbindungen. Angegeben ist der Ki-Wert der Bindungshemmung von ¹²⁵I-Fibrinogen nach Stimulierung mit ADP (10 µM). (Literatur: J.S. Bennett u. G. Vilaire, J. Clin. Invest. 64 (1979), 1393-1401; E. Kornecki et al., J. Biol. Chem. 256 (1981), 5695-5701; G.A. Marguerie et al., J. Biol. Chem. 254 (1979), 5357-5363; G.A. Marguerie et al., J. Biol. Chem. 255 (1980), 154-161).

Bei dieser Prüfung wird für die Verbindung des nachfolgenden Beispiels 1 folgendes Ergebnis erhalten:

| Beispiel | Ki (µM), ADP stimuliert |
|---|---|
| 1 | 0,42 |

Als funktioneller Test wird die Hemmung der Aggregation gelfiltrierter Human-Thrombozyten nach ADP- oder Thrombin-Stimulierung durch die erfindungsgemäßen Verbindungen gemessen. Angegeben ist der IC₅₀-Wert der Hemmung. (Literatur: G.A. Marguerie et al., J. Biol. Chem. 254 (1979), 5357-5363)

Bei dieser Prüfung wurden für die Verbindungen der nachstehenden Beispiele 1 und 2 folgende Ergebnisse erhalten:

| Beispiel | ADP-stimuliert | Thrombin-stimuliert |
|---|---|---|
| | IC₅₀(µM) | IC₅₀(µM) |
| 1 | 1,5 | 1,0 |
| 2 | 20 | 20 |

### BEISPIELE

Die Produkte wurden über Massenspektren und/oder NMR-Spektren identifiziert.

### Beispiel 1:

(1-(3-(3-(4-(Amino-imino-methyl)-phenyl)-acryloylamino)-propyl)-3-(hydroxycarbonyl-phenyl-methyl)-ureido)-essigsäure-acetat

### 1a:

### (1-(3-tert.-Butoxycarbonyl-amino-propyl)-3-(methoxycarbonyl-phenyl-methyl)-ureido)-essigsäure

2.8 g (12 mmol) (3-tert.-Butoxycarbonylaminopropyl)-glycin und 1.4 g (12 mmol) N-Ethylmorpholin werden in 300 ml Dimethylformamid gelöst. Bei 100°C werden 2.3 g (12 mmol) α-Isocyanatophenylessigsäuremethylester zugetropft. Man rührt 1 h bei 100°C, läßt auf Raumtemperatur abkühlen, engt ein und chromatographiert über Kieselgel mit Essigsäureethylester/Methanol 8:2.
Ausbeute 2.4 g

### 1b:

### (1-(3-Amino-propyl)-3-(methoxycarbonyl-phenyl-methyl)-ureido)-essigsäure-trifluoracetat

2.3 g (5.4 mmol) (1-(3-tert.-Butoxycarbonyl-amino-propyl)-3-(methoxycarbonyl-phenyl-methyl)-ureido)-essigsäure werden mit 20 ml 90%iger wässriger Trifluoressigsäure 2 h bei Raumtemperatur gerührt. Nach dem Einengen wird gefriergetrocknet.
Ausbeute: 2.3 g

### 1c:

### (3-(3-Amino-propyl)-2,5-dioxo-imidazolidin-1-yl)-phenyl-essigsäure-methylester-hydrochlorid

2.9 g (9 mmol) (1-(3-Amino-propyl)-3-(methoxycarbonyl-phenyl-methyl)-ureido)-essigsäure-trifluoracetat werden in 50 ml Methanol gelöst. Nach dem Abkühlen auf 0°C werden langsam 1.34 g (11 mmol) Thionylchlorid zugetropft. Man läßt auf Raumtemperatur erwärmen, rührt über Nacht, engt ein und kristallisiert durch Zugabe von Essigsäureethylester und Methanol.
Ausbeute: 1.99 g
Schmelzpunkt: 90°C
DCI-MS: 306 (M+H⁺)

### 1d:

### (3-(3-(3-(4-(Amino-imino-methyl)-phenyl)-acryloylamino)-propyl)-2,5-dioxo-imidazolidin-1-yl)-phenyl-essigsäure -methylester

1 g (2.93 mmol) (3-(3-Amino-propyl)-2,5-dioxo-imidazolidin-1-yl)-phenyl-essigsäure-methylester-hydrochlorid werden in 20 ml Dimethylformamid gelöst. Nach Zugabe von 0.74 g (3 mmol) 4-Amidinozimtsäurechlorid-hydrochlorid und N-Ethylmorpholin wird bei Raumtemperatur gerührt, eingeengt und der Rückstand zur Reinigung an Sephadex LH20 mit einer homogenen Mischung von Butanol/Eisessig/Wasser chromatographiert.
Ausbeute: 140 mg
FAB-MS: 478 (M+H⁺)

### 1e:

### (1-(3-(3-(4-(Amino-imino-methyl)-phenyl)-acryloylamino)-propyl)-3-(hydroxycarbonyl-phenyl-methyl)-ureido)-essig säure-acetat

78 mg (0.165 mmol) (3-(3-(3-(4-(Amino-imino-methyl)-phenyl)-acryloylamino)-propyl)-2,5-dioxo-imidazolidin-1-yl)-phenyl-essigsäure-methylester werden mit 2 ml 1N Natronlauge, 5 ml Wasser und 5 ml Methanol 20 h bei Raumtemperatur gerührt. Nach dem Einengen wird der Rückstand zur Reinigung an Sephadex LH20 mit einer homogenen Mischung von Butanol/Eisessig/Wasser chromatographiert.
Ausbeute: 23 mg
Schmelzpunkt: 240°C
FAB-MS: 482 (M+H⁺).

### Beispiel 2:

### (2-(4-(4-(Amino-imino-methyl)-benzyl)-2,5-dioxo-imidazolidin-1-yl)-ethyl)-(9-fluorenylmethoxycarbonyl)-aminoessigsäure

### 2a:

### (2-Isocyanatoethyl)-(9-fluorenylmethoxycarbonyl)-aminoessigsäure-methylester

5 g (12.8 mmol) (2-Aminoethyl)-(9-fluorenylmethoxycarbonyl)-glycinmethylester-hydrochlorid werden in 100 ml wasserfreiem Toluol suspendiert. Man leitet bei Raumtemperatur 6.3 g Phosgen ein, erhitzt auf 100°C und leitet innerhalb 6 h weiter Phosgen bei 100°C ein. Nach Zugabe von Triethylamin wird vom Salz abgetrennt, das Reaktionsgemisch eingeengt und direkt weiter umgesetzt.
Ausbeute: 4.5 g

### 2b:

### (3-(4-(Amino-imino-methyl)-phenyl)-2-(3-(2-methoxycarbonyl-methyl)-(9-fluorenylmethoxycarbonyl)-amino)-ethyl) -ureido)-propionsäure-methylester-acetat

Zu 1.5 g (5 mmol) 4-Amidinophenylalaninmethylester-di-hydrochlorid und 2.5 ml (20 mmol) N-Ethylmorpholin in 20 ml Dimethylformamid werden bei 0°C 1,9 g (5 mmol) (2-Isocyanatoethyl)-(9-fluorenylmethoxycarbonyl)-aminoessigsäure-methylester in 20 ml Dimethylformamid zugetropft. Man rührt 12 h bei Raumtemperatur, 1 h bei 50°C, versetzt mit Kaliumhydrogensulfatlösung und extrahiert mit Essigsäureethylester, trocknet die organische Phase, engt ein und chromatographiert zur Reinigung an Sephadex LH20 mit einer homogenen Mischung von Butanol/Eisessig/Wasser.
Ausbeute: 1.4 g

### 2c:

### (2-(4-(4-(Amino-imino-methyl)-benzyl)-2,5-dioxo-imidazolidin-1-yl)-ethyl)-(9-fluorenylmethoxycarbonyl)-amino essigsäure-hydrochlorid

1.2 g (1.88 mmol) (2-(4-(4-(Amino-imino-methyl)-benzyl)-2,5-dioxo-imidazolidin-1-yl)-ethyl)-(9-fluorenylmethoxy-carbonyl)-aminoessigsäure werden mit 24 ml 10%iger Salzsäure und 24 ml Essigsäure 20 Minuten auf 90°C erhitzt. Nach dem Einengen wird zur Reinigung an Sephadex LH20 mit Methanol chromatographiert.
Ausbeute: 890 mg
FAB-MS: 556 (M+H⁺)

### Beispiel 3:

### (1-(2-(4-(4-(Amino-imino-methyl)-benzyl)-2,5-dioxo-imidazolidin-1-yl)-ethyl)-3-(methoxycarbonyl-phenyl-methyl)-ureido)-essigsäure

### 3a:

### (2-(4-(4-(Amino-imino-methyl)-benzyl)-2,5-dioxo-imidazolidin-1-yl)-ethyl)-aminoessigsäure

880 mg (1.49 mmol) (2-(4-(4-(Amino-imino-methyl)-benzyl)-2,5-dioxo-imidazolidin-1-yl)-ethyl)-(9-fluorenylmethoxycarbonyl)-aminoessigsäure-hydrochlorid werden in 50 ml Dimethylformamid gelöst. Nach Zugabe von 15 ml Piperidin wird 5 Stunden bei Raumtemperatur gerührt, eingeengt, der Rückstand mit Wasser versetzt und mit Methylenchlorid extrahiert. Die Wasserphase wird auf pH 2 angesäuert, eingeengt und zur Reinigung an Sephadex LH20 mit einer homogenen Mischung von Butanol/Eisessig/Wasser chromatographiert.
Ausbeute: 285 mg
FAB-MS: 334 (M+H⁺)

### 3b:

### (1-(2-(4-(4-(Amino-imino-methyl)-benzyl)-2,5-dioxo-imidazolidin-1-yl)-ethyl)-3-(methoxycarbonyl-phenyl-methyl) -ureido)-essigsäure

Zu 100 mg (0.3 mmol) (2-(4-(4-(Amino-imino-methyl)-benzyl)-2,5-dioxo-imidazolidin-1-yl)-ethyl)-aminoessigsäure und 35 mg (0.3 mmol) N-Ethylmorpholin in 10 ml Dimethylformamid werden bei 0°C 60 mg (0.3 mmol) Isocyanatophenylglycinmethylester in 5 ml Dimethylformamid zugetropft. Man rührt 12 h bei Raumtemperatur, engt ein und chromatographiert zur Reinigung an Sephadex LH20 mit einer homogenen Mischung von Butanol/Eisessig/Wasser.
Ausbeute: 71 mg
FAB-MS: 525 (M+H⁺)

### Beispiel 4:

### N-(2-(3-(4-(Amino-imino-methyl)-phenyl)-acryloylamino)-ethyl)-N-(9-fluorenylmethoxycarbonyl)-aminoessigsäuremethylester-hydrochlorid

### 4a:

### N-(2-tert.-Butoxycarbonylaminoethyl)-N-(9-fluorenylmethoxycarbonyl)-aminoessigsäure

10.9 g (50 mmol) N-(2-tert.-Butoxycarbonylaminoethyl)-aminoessigsäure werden mit 50 ml Wasser, 11.3 g (135 mmol) Natriumhydrogencarbonat und 50 ml Tetrahydrofuran gerührt. Anschließend werden 24.3 g (72 mmol) N-(9-Fluorenylmethoxycarbonyloxy)-succinimid zugegeben. Man rührt 20 h bei Raumtemperatur, filtriert vom Salz ab, säuert an, trennt die Phasen und engt die organische Phase ein.
Ausbeute: 21.0 g (99%)

### 4b:

### N-(2-Aminoethyl)-N-(fluorenylmethoxycarbonyl)-aminoessigsäure-hydrochlorid

21.0 g (48 mmol) N-(2-tert.-Butoxycarbonylaminoethyl)-N-(fluorenylmethoxycarbonyl)-aminoessigsäure werden mit 80 ml 90%iger Trifluoressigsäure versetzt. Man rührt 3 h bei Raumtemperatur und engt im Hochvakuum ein. Der Rückstand wird in Essigsäureethylester gelöst und mit etherischer Salzsäure versetzt, das auskristallisierte Hydrochlorid wird abgesaugt und getrocknet.
Ausbeute: 13.1 g (71%)
Schmelzpunkt: 200-205°C

### 4c:

### N-(2-Aminoethyl)-N-(9-fluorenylmethoxycarbonyl)-aminoessigsäure-methylester-hydrochlorid

Zu 75 ml wasserfreiem Methanol werden bei 0°C werden 2.15 ml Thionylchlorid zugetropft. Anschließend werden 7.5 g (20 mmol) N-(2-Aminoethyl)-N-(9-fluorenylmethoxycarbonyl)-aminoessigsäure-hydrochlorid portionsweise zugegeben. Man rührt eine Stunde bei 0°C, läßt auf Raumtemperatur erwärmen und rührt über Nacht weiter. Die Reaktionslösung wird eingeengt, der Niederschlag abgesaugt und getrocknet.
Ausbeute: 6.4 g (82%)
Schmelzpunkt: 279-282°C
DCI-MS: 355 (M+H⁺)

### 4d:

### N-(2-(3-(4-(Amino-imino-methyl)-phenyl)-acryloylamino)-ethyl)-N-(9-fluorenylmethoxycarbonyl)-ammoessigsäure methylester-hydrochlorid

Zu 0.7 g (3 mmol) 4-Amidinozimtsäure-hydrochlorid 1.2 g (3 mmol) N-(2-Aminoethyl)-N-(9-fluorenylmethoxycarbonyl)-aminoessigsäure-methylester-hydrochlorid und 0.4 g (3 mmol) Hydroxybenzotriazol in 20 ml Dimethylformamid gibt man bei 0°C 0.7 g (6 mmol) N-Ethylmorpholin und 0.8 g (4 mmol) DCC. Man rührt eine Stunde bei 0°C, 20 h bei Raumtemperatur, filtriert vom ausgefallenen Harnstoff ab und engt ein. Der Rückstand wird in Essigsäureethylester aufgenommen und mit Natriumhydrogencarbonatlösung gewaschen, die organische Phase getrocknet und eingeengt. Das Produkt wird zur Reinigung an Sephadex LH20 mit einer homogenen Mischung von Butanol/Eisessig/Wasser chromatographiert.
Ausbeute: 340 mg (20%)
FAB-MS: 563.3 (M+H⁺)

### Beispiel 5:

### (1-(2-(3-(4-(Amino-imino-methyl)-phenyl)-acryloylamino)-ethyl)-3-(hydroxycarbonyl-phenyl-methyl)-ureido)-essigsäure

### 5a:

### 4-tert.-Butoxycarbonyl-amidinozimtsäure-methylester

Zu 1.95 g (8.1 mmol) 4-Amidinozimtsäure-methylesterhydrochlorid und 3.5 g (16.2 mmol) Di-tert.-butyldicarbonat in 40 ml Methylenchlorid werden bei Raumtemperatur 0.86 g (8 mmol) Natriumcarbonat in 20 ml Wasser gegeben. Nach kräftigem Rühren löst sich der Niederschlag auf. Die organische Phase wird abgetrennt, eingeengt und mit Ether kristallisiert.
Ausbeute: 1.79 g (73%)

### 5b:

### 4-tert.-Butoxycarbonyl-amidinozimtsäure-natriumsalz

730 mg (2.4 mmol) 4-tert.-Butoxycarbonyl-amidinozimtsäure-methylester werden in 50 ml Methanol gelöst. Durch Zugabe von 1N Natronlauge wird der pH-Wert auf 10 gestellt und so lange gerührt, bis alles umgesetzt ist. Die Lösung wird eingeengt und gefriergetrocknet.
Ausbeute: 748 mg

### 5c:

### (3-(2-(3-(4-(Amino-imino-methyl)-phenyl)-acryloylamino)-ethyl)-2,5-dioxoimidazolidin-1-yl)-phenyl-essigsäure -methylester-hydrochlorid

730 mg (2.34 mmol) 4-tert.-Butoxycarbonyl-amidinozimtsäure-natriumsalz werden in 50 ml Dimethylformamid gelöst und bei 0°C mit 531 mg (2.57 mmol) DCC und 316 mg (2.34 mmol) HOBt versetzt. Man rührt 50 Minuten und setzt anschließend 355 mg (2.34 mmol) (3-(2-Amino-ethyl)-2,5-dioxoimidazolidin-1-yl)-phenyl-essigsäuremethylesterhydrochlorid zu. Nach 20 h Rühren bei Raumtemperatur wird eingeengt und der Rückstand zur Reinigung an Kieselgel mit einer Mischung aus Methylenchlorid, Methanol, Eisessig und Wasser = 85:15:2:2 chromatographiert. Die Fraktionen mit der reinen Substanz werden eingeengt und gefriergetrocknet.
Ausbeute: 520 mg

### 5d:

### 1-(2-(3-(4-(Amino-imino-methyl)-phenyl)-acryloylamino)-ethyl)-3-(hydroxycarbonyl-phenyl-methyl)-ureido)-essig säure

500 mg (1 mmol) (3-(2-(3-(4-(Amino-imino-methyl)-phenyl)-acryloylamino)-ethyl)-2,5-dioxoimidazolidin-1-yl)-phenylessigsäuremethylester-hydrochlorid werden in 10 ml Methanol und 10 ml Wasser gelöst und mit 5 ml 1N Natronlauge versetzt. Man rührt über Nacht bei Raumtemperatur, engt ein und chromatographiert zur Reinigung an Sephadex LH20 mit einer homogenen Mischung von Butanol/Eisessig/Wasser.
Ausbeute: 350 mg
FAB-MS: 468.3 (M+H⁺)

### Beispiel 6:

### (3-(Carboxy-phenyl-methyl)-1-(2-(2-(4-guanidino-phenyl)-acetylamino)-ethyl)-ureido)-essigsäure

### 6a:

### (3-(2-Amino-ethyl)-2,5-dioxoimidazolidin-1-yl)-phenylessigsäure-methylester-hydrochlorid

Zu 4.1 g (10 mmol) (1-(2-tert.-Butoxycarbonyl-aminoethyl)-3-(methoxycarbonyl-phenyl-methyl)-ureido)-essigsäure in 50 ml Methanol werden bei 0°C 0.9 ml (12 mmol) Thionylchlorid zugetropft. Man läßt auf Raumtemperatur erwärmen und rührt über Nacht weiter, saugt ab und trocknet.
Ausbeute: 1.97 g (60%)

### 6b:

### (3-(2-(2-(4-Nitroguanidino-phenyl)-acetylamino)-ethyl)-2,5-dioxoimidazolidin-1-yl)-phenyl-essigsäuremethylester

270 mg (1.13 mmol) 4-Nitroguanidino-phenylessigsäure werden in 50 ml Dimethylformamid gelöst und bei 0°C mit 270 mg (1.25 mmol) DCC und 150 mg (1.13 mmol) HOBt versetzt. Man rührt 30 Minuten und setzt anschließend 370 mg (1.13 mmol) (3-(2-Amino-ethyl)-2,5-dioxoimidazolidin-1-yl)-phenyl-essigsäuremethylester-hydrochlorid und 150 µl (1.13 mmol) N-Ethylmorpholin zu. Nach 20 h Rühren bei Raumtemperatur wird eingeengt, der Rückstand mit Essigsäureethylester und Methanol versetzt und vom unlöslichen Dicyclohexylharnstoff abgesaugt. Das Filtrat wird eingeengt, mit Methylenchlorid versetzt und die Lösung mit Natriumhydrogencarbonatlösung und Kaliumhydrogensulfatlösung gewaschen, getrocknet und eingeengt.
Ausbeute: 540 mg

### 6c:

### (3-(Carboxy-phenyl-methyl)-1-(2-(2-(4-guanidino-phenyl)-acetylamino)-ethyl)-ureido)-essigsäure

380 mg (0.74 mmol) (3-(2-(2-(4-Nitroguanidino-phenyl)-acetylamino)-ethyl)-2,5-dioxoimidazolidin-1-yl)-phenylessigsäuremethylester werden in 5 ml Methanol und 5 ml Wasser gelöst. Nach Zugabe von 1.5 ml (1.5 mmol) 1N Natronlauge wird 5 h bei Raumtemperatur gerührt, eingeengt und gefriergetrocknet. Der Rückstand wird in 40 ml Methanol und 40 ml Wasser gelöst, mit 50 mg 10%-Palladium auf Kohle versetzt und bei Raumtemperatur hydriert. Nach dem Einengen wird zur Reinigung an Sephadex LH20 mit einer homogenen Mischung von Butanol/Eisessig/Wasser chromatographiert.
Ausbeute: 200 mg (57%)
FAB-MS: 471.3 (M+H⁺)

### Beispiel 7:

### (3-(Carboxy-phenyl-methyl)-1-(3-(4-guanidino-benzoylamino)-propyl)-ureido)-essigsäure

### 7a:

### (3-(3-(4-Guanidino-benzoyl-amino)-propyl)-2,5-dioxoimidazolidin-1-yl)-phenyl-essigsäuremethylester-hydrochlorid

384 mg (1.14 mmol) 4-Guanidinobenzoesäure-4-nitrophenylester, 390 mg (1.14 mmol) (3-(3-Amino-propyl)-2,5-dioxoimidazolidin-1-yl)-phenyl-essigsäuremethylester-hydrochlorid, 55 mg (0.4 mmol) HOBt und 140 mg (1.2 mmol) N-Ethylmorpholin werden in 10 ml Dimethylformamid 5 h bei Raumtemperatur gerührt. Nach dem Einengen wird zur Reinigung an Kieselgel mit einer Mischung aus Methylenchlorid, Methanol, Eisessig und Wasser = 85:15:2:2 chromatographiert. Die Fraktionen mit der reinen Substanz werden eingeengt und gefriergetrocknet.
Ausbeute: 385 mg (67%)

### 7b:

### (3-(Carboxy-phenyl-methyl)-1-(3-(4-guanidino-benzoylamino)-propyl)-ureido)-essigsäure

380 mg (0.76 mg) (3-(3-(4-Guanidino-benzoyl-amino)-propyl)-2,5-dioxoimidazolidin-1-yl)-phenyl-essigsäuremethylester-hydrochlorid werden in 10 ml Methanol und 10 ml Wasser gelöst und mit 2.28 ml 1N Natronlauge versetzt. Man rührt über Nacht bei Raumtemperatur, engt ein und chromatographiert zur Reinigung an Sephadex LH20 mit einer homogenen Mischung von Butanol/Eisessig/Wasser.
Ausbeute: 276 mg (77%)
FAB-MS: 471.4 (M+H⁺)

## Patentansprüche

1. Verbindungen der allgemeinen Formel I
R¹-(A)ₐ-(B)_{b}-(D)_{c}-(CH₂)ₘ-N(R²)-(CH₂)ₙ-R³ (I)
worin
A einen zweiwertigen Rest aus der Reihe Methylen, Ethylen, Trimethylen, Tetramethylen, Cyclohexylen, Phenylen, Phenylenmethyl, Phenylenethenyl und Phenylenmethylen bedeutet;
B einen zweiwertigen Rest aus der Reihe Methylen, Ethylen, Trimethylen, Tetramethylen, Vinylen, Phenylen, 2,5-Dioxo-imidazolidin-1,4-diyl, 5-Oxo-2-thioxoimidazolidin-1,4-diyl, 2,5-Dioxo-pyrrolidin-1,4-diyl und 2,4-Dioxo-oxazolidin-3,5-diyl bedeutet;
D einen zweiwertigen Rest aus der Reihe Carbonylimino, Iminocarbonyl und Iminocarbonylimino bedeutet;
R¹ -CH₂-NH-X, -C(=NH)-NH-X¹ oder -NH-C(-NX¹)-NH-X^{1'} bedeutet;
X, X¹ und X^{1'} unabhängig voneinander Wasserstoff, (C₁-C₆)-Alkylcarbonyl, (C₁-C₆)-Alkoxycarbonyl oder (C₁-C₈)-Alkylcarbonyloxy-(C₁-C₆)-alkoxycarbonyl bedeuten;
R² -COR⁴, -COOR⁴, -CO-NH-R⁴ oder -CS-NH-R⁴ bedeutet;
R³ -COOH oder -COO-(C₁-C₁₈)-Alkyl bedeutet;
R⁴ (C₁-C₆)-Alkyl oder (C₆-C₁₄)-Aryl-(C₁-C₈)-alkyl bedeutet oder im Falle, daß R² für -CO-NH-R⁴ oder -CSNH-R⁴ steht, -NH-R⁴ für einen α-Aminosäurerest oder dessen ω-Amino-(C₂-C₈)-alkylamid steht;
a, b und c 0 oder 1 bedeuten, aber nicht alle gleichzeitig 0 sein können; und
m für 2 oder 3 und n für 1 steht;
sowie deren physiologisch verträgliche Salze.

2. Verbindungen gemäß Anspruch 1, dadurch gekennzeichnet, daß für -NH-R⁴ stehende α-Aminosäurereste der Valin-, Lysin-, Phenylalanin-, Phenylglycin- oder der Tryptophan-Rest sind.

3. Verbindungen gemäß Anspruch 1, dadurch gekennzeichnet, daß das ω-Amino-(C₂-C₈)-alkylamid das 4-Amino-butylamid ist.

4. Verfahren zur Herstellung von Verbindungen der allgemeinen Formel I der Ansprüche 1 bis 3, dadurch gekennzeichnet, daß man eine Fragmentkondensation einer Verbindung der allgemeinen Formel II
R¹ - (A)ₐ - (B)_{b} - Y (II)
mit einer Verbindung der allgemeinen Formel III
Z - (CH₂)ₘ - N(R²) - (CH₂)ₙ - R³ (III)
ausführt, wobei die Reste A, B, R¹, R² und R³ sowie a, b, m und n wie in den Ansprüchen 1 bis 3 angegeben definiert sind und Y für Hydroxycarbonyl, Alkoxycarbonyl oder ein aktiviertes Carbonsäurederivat und Z für Amino steht oder worin Y für Amino und Z für Hydroxycarbonyl, Alkoxycarbonyl oder ein aktiviertes Carbonsäurederivat steht.

5. Verbindung der allgemeinen Formel I der Ansprüche 1 bis 3 zur Anwendung als Hemmstoff der Thrombozytenaggregation, der Metastasierung von Karzinomzellen oder der Osteoclastenbindung an die Knochenoberflächen.

6. Pharmazeutisches Präparat, dadurch gekennzeichnet, daß es eine oder mehrere Verbindungen der allgemeinen Formel I der Ansprüche 1 bis 3 oder ein physiologisch verträgliches Salz davon als Wirkstoff zusammen mit pharmazeutisch annehmbaren Träger- und Zusatzstoffen und gegebenenfalls noch einen oder mehrere andere pharmakologische Wirkstoffe enthält.

7. Verfahren zur Herstellung eines pharmazeutischen Präparates, enthaltend eine oder mehrere Verbindungen der allgemeinen Formel I der Ansprüche 1 bis 3 oder ein physiologisch verträgliches Salz davon, dadurch gekennzeichnet, daß man diese zusammen mit pharmazeutisch annehmbaren Träger- und Zusatzstoffen und gegebenenfalls noch einem oder mehreren anderen pharmakologischen Wirkstoffen in eine geeignete Darreichungsform bringt.

## Claims

1. Compounds of the general formula I
R¹-(A)ₐ-(B)_{b}-(D)_{c}-(CH₂)ₘ-N(R²)-(CH₂)ₙ-R³ (I)
in which
A denotes a divalent radical from the group comprising methylene, ethylene, trimethylene, tetramethylene, cyclohexylene, phenylene, phenylenemethyl, phenyleneethenyl and phenylenemethylene;
B denotes a divalent radical from the group comprising methylene, ethylene, trimethylene, tetramethylene, vinylene, phenylene, 2,5-dioxoimidazolidine-1,4-diyl, 5-oxo-2-thioxoimidazolidine-1,4-diyl, 2,5-dioxopyrrolidine-1,4-diyl and 2,4-dioxooxazolidine-3,5-diyl;
D denotes a divalent radical from the group comprising carbonylimino, iminocarbonyl and iminocarbonylimino;
R¹ denotes -CH₂-NH-X, -C(=NH)-NH-X¹ or -NH-C (=NX¹) -NH-X^{1'};
X, X¹ and X^{1'} independently of one another denote hydrogen, (C₁-C₆) -alkylcarbonyl, (C₁-C₆) -alkoxycarbonyl or (C₁-C₈)-alkylcarbonyloxy-(C₁-C₆)-alkoxycarbonyl;
R² denotes -COR⁴, -COOR⁴, -CO-NH-R⁴ or -CS-NH-R⁴;
R³ denotes -COOH or -COO-(C₁-C₁₈)-alkyl;
R⁴ denotes (C₁-C₆)-alkyl or (C₆-C₁₄)-aryl-(C₁-C₈)-alkyl, or, in the case where R² represents -CO-NH-R⁴ or -CSNH-R⁴, -NH-R⁴ represents an α-amino acid residue or its ω-amino-(C₂-C₈)-alkyl amide;
a, b and c denote 0 or 1, but cannot all simultaneously be 0; and
m represents 2 or 3 and n represents 1;
as well as physiologically tolerated salts thereof.

2. Compounds according to Claim 1, characterized in that α-amino acid residues representing -NH-R⁴ are the valine, lysine, phenylalanine, phenylglycine or tryptophan residue.

3. Compounds according to Claim 1, characterized in that the ω-amino-(C₂-C₈)-alkyl amide is the 4-aminobutyl amide.

4. Process for preparing compounds of the general formula I of Claims 1 to 3, characterized in that a fragment condensation of a compound of the general formula II
R¹-(A)ₐ-(B)_{b}-Y (II)
with a compound of the general formula III
Z-(CH₂)ₘ-N(R²)-(CH₂)ₙ-R³ (III)
is carried out, where the radicals A, B, R¹, R² and R³, as well as a, b, m and n, are defined as indicated in Claims 1 to 3, and Y represents hydroxycarbonyl, alkoxycarbonyl or an activated carboxylic acid derivative, and Z represents amino, or in which Y represents amino, and Z represents hydroxycarbonyl, alkoxycarbonyl or an activated carboxylic acid derivative.

5. Compound of the general formula I of Claims 1 to 3 for use as an inhibitor of blood-platelet aggregation, the formation of metastases by carcinoma cells or the binding of osteoclasts to bone surfaces.

6. Pharmaceutical preparation, characterized in that it contains one or more compounds of the general formula I of Claims 1 to 3, or a physiologically tolerated salt thereof, as the active compound, together with pharmaceutically acceptable excipients and additives and, where appropriate, one or more different pharmacological active compounds in addition.

7. Process for preparing a pharmaceutical preparation containing one or more compounds of the general formula I of Claims 1 to 3, or a physiologically tolerated salt thereof, characterized in that these, together with pharmaceutically acceptable excipients and additives and, where appropriate, one or more different pharmacological active compounds in addition, are brought into a suitable form for administration.

## Revendications

1. Composés de formule générale I
R¹-(A)ₐ-(B)_{b}-(D)_{c}-(CH₂)ₘ-N(R²)-(CH₂)ₙ-R³ (I)
dans laquelle
A représente un radical divalent de la série: méthylène, éthylène, triméthylène, tétraméthylène, cyclohexylène, phénylène, phénylène méthyle, phénylène éthényle et phénylène méthylène ;
B représente un radical divalent de la série: méthylène, éthylène, triméthylène, tétraméthylène, vinylène, phénylène, 2,5-dioxo-imidazolidin-1,4-diyle, 5-oxo-2-thioxoimidazolidin-1,4-diyle, 2,5-dioxo-pyrrolidin-1,4-diyle et 2, 4-dioxo-oxazolidin-3, 5-diyle;
D représente un radical divalent de la série: carbonylimino, iminocarbonyle et iminocarbonylimino;
R¹ représente -CH₂-NH-X, -C(=NH)-NH-X¹ ou -NH-C(=NX¹)-NH-X^{1'};
X, X¹ et X^{1'} représentent, indépendamment les uns des autres, un hydrogène, un alkyl(en C₁ - C₆)carbonyle, un alcoxy(en C₁ - C₆)carbonyle ou un alkyl(en C₁ - C₈) carbonyloxy-alcoxy (en C₁ - C₆)carbonyle;
R² représente -COR⁴, -COOR⁴, -CO-NH-R⁴ ou -CS-NH-R⁴;
R³ représente -COOH ou -COO-alkyle(en C₁ - C₁₈);
R⁴ représente un alkyle en C₁ - C₆ ou un aryl(en C₆ - C₁₄)-alkyle(en C₁ - C₈), ou bien, dans le cas où R² est -CO-NH-R⁴ ou -CS-NH-R⁴, -NH-R⁴ est un radical α-aminoacide ou un ω-amino-alkyl(en C₂ - C₈)amide de celui-ci;
a, b et c représentent 0 ou 1, mais ne peuvent pas être tous en même temps égaux à 0; et
m est égal à 2 ou 3, et n à 1;
ainsi que les sels physiologiquement compatibles de ceux-ci.

2. Composés selon la revendication 1, caractérisés en ce que les radicaux α-aminoacide qui conviennent pour -NH-R⁴ sont un radical valine, lysine, phénylalanine, phénylglycine ou tryptophane.

3. Composés selon la revendication 1, caractérisés en ce que le ω-amino-alkyl(en C₂ - C₈)-amide est le 4-amino-butylamide.

4. Procédé de fabrication des composés de formule générale I des revendications 1 à 3, caractérisé en ce qu'on réalise une condensation des fragments d'un composé de formule générale II
R¹- (A)ₐ- (B)_{b}-Y (II)
avec un composé de formule générale III
Z- (CH₂)ₘ-N(R²) - (CH₂)ₙ-R³ (III)
les radicaux A, B, R¹, R² et R³ ainsi que a, b, m et n étant définis comme indiqué dans les revendications 1 à 3, et Y étant un hydroxycarbonyle, un alcoxycarbonyle ou un dérivé d'acide carboxylique activé, et Z étant un groupe amino, ou bien dans lesquelles Y est un groupe amino et Z un hydroxycarbonyle, un alcoxycarbonyle ou un dérivé d'acide carboxylique activé.

5. Composé de formule générale I selon les revendications 1 à 3, pour une utilisation comme agent inhibiteur de l'agrégation des thrombocytes, de la dissémination métastasique des cellules d'un carcinome ou de la fixation des ostéoclastes à la surface des os.

6. Préparation pharmaceutique, caractérisée en ce que qu'elle contient un ou plusieurs composés de formule générale I selon les revendications 1 à 3, ou un sel physiologiquement compatible de ceux-ci, comme ingrédient actif, conjointement avec des véhicules et des additifs pharmaceutiquement acceptables et, le cas échéant, également un ou plusieurs ingrédients pharmacologiquement actif.

7. Procédé de fabrication d'une préparation pharmaceutique contenant un ou plusieurs composés de formule générale I selon les revendications 1 à 3, ou un sel physiologiquement compatible de ceux-ci, caractérisé en ce qu'on associe ceux-ci à des véhicules et des additifs pharmaceutiquement acceptables et, le cas échéant, également avec un ou plusieurs autres ingrédients pharmaceutiquement,actifs sous une forme administrable adaptée.
